# DEMANDE DE BREVET EUROPEEN

(11) **EP 0 624 575 A1**
(43) Date de publication de la demande: **17.11.1994**
(21) Numéro de dépôt: 94401050.3
(22) Date de dépôt: 11.05.1994
(51) Int. Cl.: C07D 209/42, A61K 31/40

(54) **Indoles substitués et les compositions pharmaceutiques qui les contiennent**

(30) Priorité: 12.05.1993 FR 9305686
(71) Demandeur: ADIR ET COMPAGNIE, F-92415 Courbevoie Cédex (FR)
(72) Inventeur: Caubere, Paul, F-54000 Nancy (FR); Jamart-Gregoire, Brigitte, F-54500 Vandoeuvre les Nancy (FR); Caubere, Catherine, Bâtiment C., F-54000 Nancy (FR); Bizot-Espiard, Jean-Guy, F-75015 Paris (FR); Renard, Pierre, F-78000 Versailles (FR); Adam, Gérard, F-78600 Le Mesnil le Roi (FR)

(57) **Abrégé**

Composés de formule générale (I) : 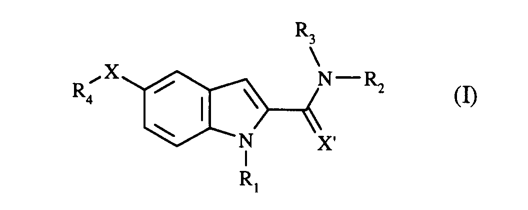
dans laquelle :
- X représente O ou S,
- X' représente O, S ou H₂,
- et R₁, R₂, R₃ et R₄ sont tels que définis dans la description.

Ces composés trouvent leur application en thérapeutique dans le traitement ou la prévention des affections dues ou reliées à des phénomènes de peroxydation et notamment les désordres ischémiques cérébraux, rénaux ou cardiaques et les maladies métaboliques notamment l'athérome et l'artériosclérose, ainsi que l'inflammation.

## Description

La présente invention concerne de nouveaux indoles substitués, leur procédé de préparation et les compositions pharmaceutiques qui les contiennent.

La littérature fournit quelques exemples d'indoles substitués en position 2 : les travaux de E. Fernandez-Alvarez *et al*., dans le brevet ES-2013391, montrent la préparation de dérivés alléniques et acétyléniques de 2-aminométhyl-5-méthoxyindoles à partir des carboxamides correspondants. Dans Eur. J. Med. Chem., 25, 257-265, (1990), les mêmes auteurs montrent, pour ces mêmes dérivés, leur activité inhibitrice des formes A et B de la monoamine oxydase (MAO).

La demanderesse a découvert que de nouveaux indoles substitués en position 2 sont doués de propriétés pharmacologiques très intéressantes de par leur pouvoir inhibiteur spécifique vis-à-vis de l'oxydation des LDL (Low Density Lipoproteins) humaines et des lipides membranaires.

Plus précisément, l'invention concerne de nouveaux indoles, de formule générale (I) : 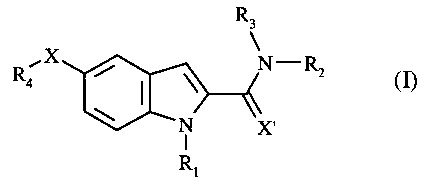
dans laquelle :
- R₁ est choisi parmi l'hydrogène et un radical alkyle linéaire ou ramifié, éventuellement substitué par un ou plusieurs groupements R₅, et comportant de 1 à 5 atomes de carbone,
- R₂ est choisi parmi l'hydrogène, un radical alkyle linéaire ou ramifié, éventuellement substitué par un ou plusieurs groupements R₅, et comportant de 1 à 12 atomes de carbone, et un radical cycloaliphatique comportant de 3 à 8 atomes de carbone éventuellement substitué par un ou plusieurs groupement R₅,
- R₃ est choisi parmi:
   * un radical cycloaliphatique comportant de 3 à 8 atomes de carbone,
   * un radical aryle, choisi parmi le radical phényle et le radical naphtyle,
   * et un radical hétéroaryle choisi parmi le radical furyle, thiényle, thiazolyle, pyrrolyle, imidazolyle, pyridyle, pyrimidinyle, pyrazolyle, pyrazinyle, pyridazinyle, quinolyle, isoquinolyle, indolyle, benzofuranyle, benzo[b]thiényle et benzimidazolyle,
   chacun des radicaux cycloaliphatiques, aryles et hétéroaryles pouvant être éventuellement substitués par un ou plusieurs groupements R₅,
- ou bien R₂ et R₃ forment ensemble avec l'atome d'azote auquel ils sont reliés un radical choisi parmi :
   a) le radical de formule (a) : 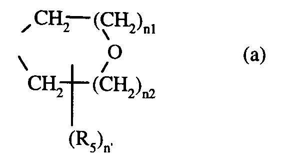
   b) le radical de formule (b) : 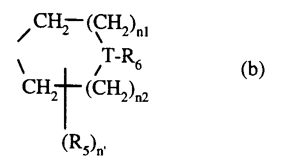
   c) et le radical de formule (c) : 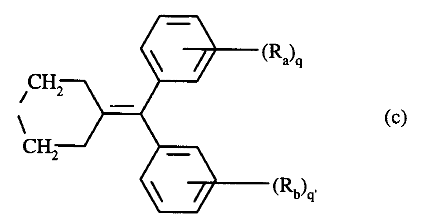
- R₄ est choisi parmi l'hydrogène, un radical alkyle linéaire ou ramifié, éventuellement substitué par un ou plusieurs groupements R₅, et comportant de 1 à S atomes de carbone, un radical aryle, hétéroaryle, choisis parmi ceux décrits pour le radical R₃, un radical arylalkyle et un radical hétéroarylalkyle dans lequel les radicaux aryle et hétéroaryle sont tels que définis précédemment et la chaîne alkyle comporte de 1 à 5 atomes de carbone en chaîne droite ou ramifiée, chacun étant éventuellement substitué par un ou plusieurs groupements R₅,
- R₅ est choisi parmi un halogène, un groupement hydroxy, nitro, cyano, alkyle, alkoxy, acyle, carboxy, alcoxycarbonyle, carboxamido, halogénoalkyle, amino, alkylamino et dialkylamino, les chaînes alkyles des groupements alkyles, alkoxy, acyles, alcoxycarbonyles, carbamoyles, halogénoalkyles, alkylamino et dialkylamino comportant de 1 à 5 atomes de carbone en chaîne droite ou ramifiée,
- R₆ représente soit l'hydrogène, soit un radical choisi parmi :
   α) le radical 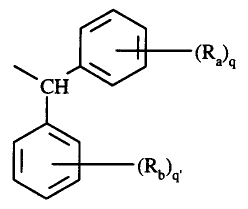 β) et le radical 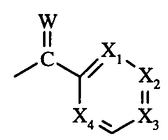
- X₁, X₂, X₃ et X₄ représentent indépendamment l'un de l'autre l'azote, le groupement CH ou le groupement C-Rₐ,
- Rₐ et R_{b}, identiques ou différents, sont choisis indépendamment l'un de l'autre parmi un halogène, un radical hydroxy, alkyle, alkoxy et halogénoalkyle, chacun contenant de 1 à 5 atomes de carbone en chaîne droite ou ramifiée,
- T est choisi parmi le groupement CH ou l'atome d'azote,
- W est choisi parmi O ou H₂,
- n₁ prend les valeurs 0, 1, 2, ou 3,
- n₂, dépendant de n₁, prend les valeurs entières comprises inclusivement entre 0 et (3-n₁),
- n' prend les valeurs de 0 à (n₁+n₂+2) (bornes incluses),
- m est choisi parmi 0, 1, 2, 3 et 4,
- q et q' prennent indépendamment l'un de l'autre les valeurs de 0 à 5 (bornes incluses),
- X est choisi parmi O ou S,
- X' est choisi parmi O, S ou H₂,
   avec les restrictions suivantes:
   - lorsque R₂ représente l'hydrogène alors R₃ ne peut pas représenter un radical phényle substitué en position 4 par une chaîne de nature oxy-2-hydroxy (ou alkoxy)-3-aminopropyle, ni un radical phényle substitué en position 2 par un radical carboxy,
   - lorsque R₂ représente l'hydrogène, X le soufre et R₄ le radical para-nitrophényle, alors R₃ ne peut pas représenter un radical aryle éventuellement substitué,
   - lorsque R₂ et R₃ forment ensemble avec l'atome d'azote auquel ils sont reliés un radical choisi parmi ceux de formules (a), (b) et (c) tels que définis précédemment, alors -X-R₄ ne peut représenter un radical -O-alkyle tel que défini précédemment,

leurs éventuels stéréoisomères, N-oxydes et sels d'addition à un acide ou une base pharmaceutiquement acceptables.

Parmi les acides pharmaceutiquement acceptables que l'on peut utiliser pour former un sel d'addition avec les composés de l'invention, on peut citer à titre d'exemples non limitatifs, les acides chlorhydrique, phosphorique, sulfurique, tartrique, citrique, maléique, fumarique, alkylsulfonique et camphorique.

Parmi les bases pharmaceutiquement acceptables que l'on peut utiliser pour former un sel d'addition avec les composés de l'invention, on peut citer, à titre d'exemples non limitatifs, l'hydroxyde de sodium ou de potassium, la diéthylamine, la triéthylamine, l'éthanolamine, la diéthanolamine, l'arginine et la lysine.

La présente invention s'étend également au procédé de préparation des composés de formule (I), caractérisé en ce que le produit de départ est l'acide de formule (II) : 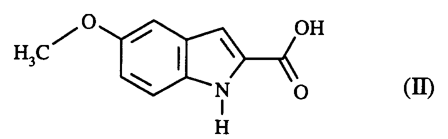
qui est transformé en chlorure d'acyle correspondant par un réactif approprié, par exemple le pentachlorure de phosphore, dans un solvant polaire, par exemple l'éther, puis est soumis à l'action d'une amine secondaire de formule (III) : 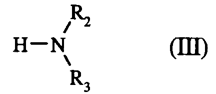
dans laquelle R₂ et R₃ sont tels que définis pour la formule (I),
afin d'obtenir l'amide de formule (IV) : 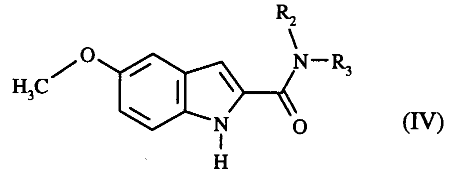
dans laquelle R₂ et R₃ sont tels que définis précédemment,
dont l'atome d'azote de l'indole est éventuellement alkylé par action successive d'un agent de déprotonation, comme l'hydrure de sodium, puis d'un agent alkylant de formule B-R'₁ dans laquelle B représente un atome d'halogène ou un groupement sulfate et R'₁ représente un radical alkyle linéaire ou ramifié, éventuellement substitué par un ou plusieurs groupements R₅, et comportant de 1 à 5 atomes de carbone, dans un solvant approprié, tel que le diméthylformamide, pour fournir les composés de formule (IV') : 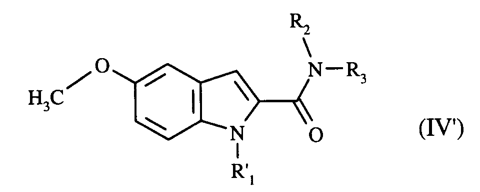
dans laquelle R'₁, R₂ et R₃ sont tels que définis précédemment,
l'ensemble des composés de formules (IV) et (IV') formant l'ensemble des composés de formule (Va) : 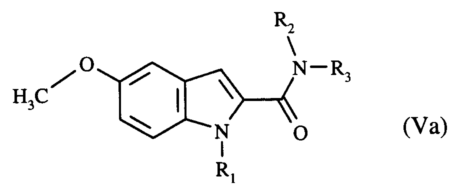
dans laquelle R₁, R₂ et R₃ sont tels que définis précédemment,
cas particulier des composés de formule (I), dans laquelle -X-R₄ représente le groupe méthoxy,
les composés de formule (Va) pouvant, si on le souhaite, être soumis à une réaction de O-déméthylation selon la méthode décrite par Fujita *et al*. (J. Org. Chem., 45, 4275, (1980)) par action d'un acide de Lewis et d'un alkylthiol, afin d'obtenir les composés de formule (Vb) : 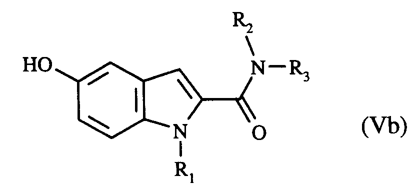
dans laquelle R₁, R₂ et R₃ sont tels que définis précédemment,
réaction conduisant également, selon les conditions opératoires, et en utilisant un alkylthiol de formule R'₄-SH, dans laquelle R'₄ prend toutes les valeurs possibles pour R₄, l'hydrogène excepté, aux composés de formule (Vc): 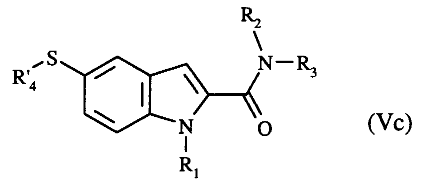
dans laquelle les substituants R₁, R₂, R₃ et R'₄ sont tels que définis précédemment,
les composés de formule (Vd) : 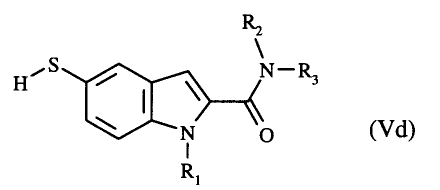
dans laquelle R₁, R₂ et R₃ sont tels que définis précédemment,
étant obtenus par débenzylation, par réduction chimique ou catalytique, des composés de formule (Vc') : 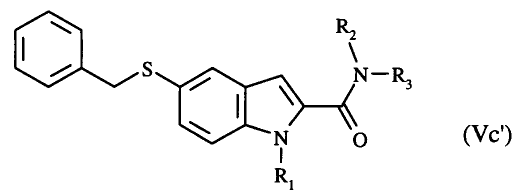
cas particulier des composés de formule (Vc) dans laquelle R'₄ représente le radical benzyle,
les composés de formule (Vb) étant éventuellement engagés dans une réaction classique d'éthérification pour fournir les composés de formule (Ve) : 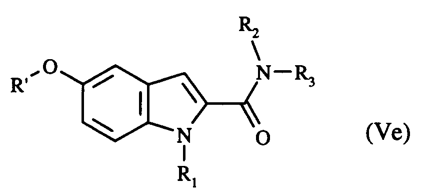
dans laquelle R₁, R₂ et R₃ sont tels que définis précédemment et R' représente un radical allyle linéaire ou ramifié, éventuellement substitué, contenant de 2 à 5 atomes de carbone, un radical aryle, arylalkyle, hétéroaryle ou hétéroarylalkyle, choisis parmi ceux décrits pour R₄,
l'ensemble des composés de formules (Va), (Vb), (Vc), (Vd) et (Ve) formant l'ensemble des composés de formule (V): 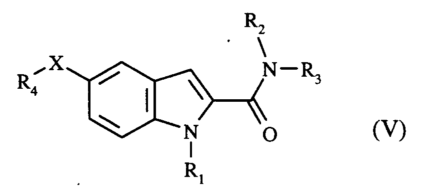
dans laquelle X, R₁, R₂, R₃ et R₄ sont tels que définis précédemment,
cas particulier des composés de formule (I) dans laquelle X' représente l'oxygène, qui sont soit:
- transformés, par traitement par le réactif de Lawesson, en thioamide de formule (V'): 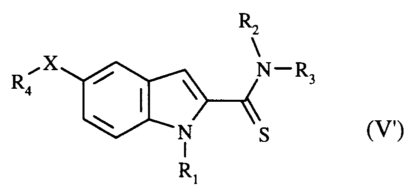 dans laquelle X, R₁, R₂, R₃ et R₄ sont tels que définis précédemment,
   cas particulier des composés de formule (I) dans laquelle X' représente le soufre,
- soit réduits, à l'aide d'un agent réducteur, tel que l'hydrure double de lithium et d'aluminium, en solvant anhydre, tel que l'éther éthylique, en composés de formule (V'') : 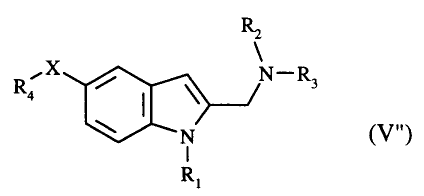 dans laquelle X, R₁, R₂, R₃ et R₄ sont tels que définis précédemment,
   cas particulier des composés de formule (I) dans laquelle X' représente le groupement H₂,

l'ensemble des composés de formules (V), (V') et (V'') formant l'ensemble des composés de formule (I) qui sont purifiés et éventuellement séparés en leurs stéréoisomères par une technique classique de séparation et qui sont, si on le désire, transformés en leurs sels d'addition à un acide ou à une base pharmaceutiquement acceptables.

La réaction d'alkylation de l'atome d'azote décrite pour les composés de formule (IV) peut également être effectuée directement sur le composé de départ de formule (II). Dans cette éventualité, le composé obtenu est le composé de formule (VII): 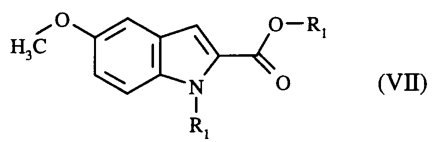
dans laquelle R₁ est tel que défini précédemment,
qui est ensuite saponifié par l'action d'une base, telle que l'hydroxyde de potassium, pour donner le composé de formule (VIII) : 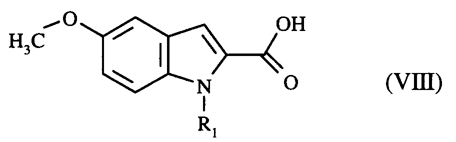
dans laquelle R₁ est tel que défini précédemment,
qui est ensuite traité de façon analogue au composé de formule (II) pour conduire aux composés de formule (I).

Les composés de formule (IX) : 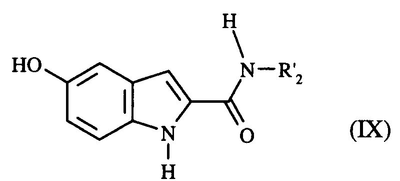
cas particulier des composés de formule (I) dans laquelle R₁ représente l'hydrogène, R'₂ représente un radical aryle tel que défini précédemment, R₃ et R₄ représentent chacun l'hydrogène et X et X' représentent chacun l'oxygène, sont obtenus à partir de l'acide 5-hydroxy-indole-2-carboxylique qui est transformé en son chlorure d'acyle correspondant par action de chlorure de thionyle, celui-ci étant ensuite traité par une amine de formule (X) : 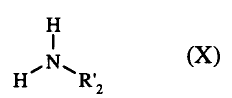
dans laquelle R'₂ est tel que défini précédemment.

Les composés de la présente invention possèdent de façon surprenante des propriétés anti-oxydantes très importantes. Les études pharmacologiques ont notamment montré que ces composés sont doués d'activités protectrices remarquables et spécifiques vis-à-vis des peroxydations des lipoprotéines de faible densité (LDL), ainsi que des lipides membranaires.

Les composés de l'invention ont également montré une activité anti-inflammatoire et sont doués d'un pouvoir anti-agrégant plaquettaire.

Les composés de l'invention présentent donc une action particulièrement nouvelle et bénéfique dans les affections où intervient une peroxydation des lipides membranaires. Ainsi, ces nouveaux composés peuvent être utilisés dans le traitement ou la prévention des affections dues ou reliées à de tels phénomènes de peroxydation et notamment les désordres ischémiques cérébraux, rénaux ou cardiaques et les maladies métaboliques notamment l'athérome et l'artériosclérose, ainsi que l'inflammation.

La présente invention a également pour objet les compositions pharmaceutiques contenant un dérivé de formule (I), ou un de ses sels d'addition à un acide ou une base pharmaceutiquement acceptables, seul ou en combinaison avec un ou plusieurs excipients inertes et non toxiques. Parmi les compositions pharmaceutiques selon l'invention, on pourra citer plus particulièrement celles qui conviennent à l'administration orale, parentérale, nasale, rectale, perlinguale, oculaire ou pulmonaire et notamment les préparations injectables, les aérosols, les gouttes oculaires ou nasales, les comprimés simples, pelliculés ou dragéifiés, les gélules, les capsules, les crèmes, pommades, gels dermiques, les suppositoires,....

La posologie utile varie selon l'âge et le poids du patient, la voie d'administration, la nature de l'affection et des traitements éventuels associés et s'échelonne entre 0,5 mg et 2 grammes par 24 heures.

Les exemples suivants illustrent l'invention sans toutefois la limiter en aucune façon.

Les matières premières sont disponibles ou préparées à partir de modes opératoires connus.

### EXEMPLE 1 : N-Méthyl-N-phényl-(5-méthoxyindol-2-yl)carboxamide

1 g (5,23 mmol) d'acide S-méthoxy-indole-2-carboxylique est ajouté à 60 ml d'éther éthylique anhydre, puis 1,64 g (7,85 mmol) de pentachlorure de phosphore. Lorsque la solution est devenue limpide, le solvant est évaporé sous vide. Le résidu est repris avec 50 ml d'éther anhydre, qui est ensuite évaporé puis, la même opération est effectuée deux fois avec 50 ml de trichlorométhane. Le précipité obtenu est à nouveau dissous dans 50 ml d'éther anhydre. La solution est refroidie à 0°C dans un bain de glace, puis sont ajoutés goutte à goutte 1,12 g (10,46 mmol) de N-méthylaniline dans 15 ml de dioxane sec. La solution est agitée pendant une nuit à température ambiante, le solvant est ensuite évaporé sous vide et le précipité est repris dans 50 ml de trichlorométhane, lavé avec une solution d'acide chlorhydrique diluée (1N), puis quatre fois à l'eau. La phase organique est alors séchée sur sulfate de magnésium, puis le solvant est évaporé sous vide. Le précipité obtenu est ensuite lavé plusieurs fois avec de l'éther de pétrole et recristallisé dans l'éthanol.
Rendement : 86 %.
Point de fusion : 194°C.

### EXEMPLE 2: N-Méthyl-N-phényl-(5-méthoxy-1-méthylindol-2-yl)carboxamide.

174 mg (7,6 mmol) d'hydrure de sodium sont ajoutés à 10 ml de diméthylformamide refroidis à 0°C, sous atmosphère inerte. 1 g (3,8 mmol) de composé obtenu à l'exemple 1 sont alors ajoutés goutte à goutte. Après retour à température ambiante, le milieu est agité encore pendant 5 minutes avant d'ajouter lentement 1,43 g (11,4 mmol) de sulfate de diméthyle. Après 1 h 30, la solution devenue limpide est hydrolysée et traitée par une solution aqueuse à 10 % d'ammoniaque, puis extraite à l'éther éthylique. Après traitement habituel de la phase organique, le produit attendu est recristallisé dans un mélange acétate d'éthyle / éther de pétrole (50/50).
Rendement : 84 %.
Point de fusion : 94°C.

### EXEMPLE 3: N-Méthyl-N-phényl-(5-hydroxy-1-méthylindol-2-yl)carboxamide

A un mélange refroidi à 0°C de 360 mg (2,7 mmol) de chlorure d'aluminium et de 2,2 g (36 mmol) d'éthanethiol sont ajoutés 500 mg (1,8 mmol) de composé obtenu à l'exemple 2, dissous dans 5 ml de chlorure de méthylène préalablement distillé sur pentoxyde de phosphore. Après 1 heure d'agitation à 0°C, 1,5 équivalents de chlorure d'aluminium et 20 équivalents d'éthanethiol sont à nouveau ajoutés. Après 2 heures à 0°C, le mélange est jeté sur de la glace et acidifié jusqu'à pH = 2 avec une solution 1N d'acide chlorhydrique. Après extraction au dichlorométhane, traitement de la phase organique, et purification par chromatographie sur colonne de silice, on obtient l'indole phénolique attendu.
Rendement : 50 %.
Point de fusion : 139°C.

### EXEMPLE 4: N-Phényl-(5-méthoxy-1-méthylindol-2-yl)carboxamide

500 mg (21 mmol) d'hydrure de sodium sont ajoutés à 10 ml de diméthylformamide à 0°C, puis 1 g (5,23 mmol) d'acide 5-méthoxyindole-2-carboxylique dissous dans 20 ml de diméthylformamide. Après retour à température ambiante, le mélange est agité pendant encore 5 minutes, puis 2,3 g (18,3 mmol) de sulfate de diméthyle sont ajoutés. Le milieu réactionnel est ensuite hydrolysé, traité par une solution à 10 % d'ammoniaque puis extrait à l'éther. Après traitement habituel de la phase organique, on obtient un solide correspondant au 5-méthoxy-1-méthylindole-2-carboxylate de méthyle utilisé brut pour la saponification. A cet effet, 1 g (4,9 mmol) d'ester dans 30 ml d'une solution d'hydroxyde de potassium à 10 % dans l'éthanol est porté à reflux pendant 1 heure puis refroidi. Après extraction à l'éther, acidification par une solution à 10 % d'acide chlorhydrique et traitement habituel de la phase organique, le solide obtenu est traité par l'aniline comme décrit dans l'exemple 1. L'amide attendue est recristallisée dans l'éthanol.
Rendement : 70 %.
Point de fusion : 200°C.

### EXEMPLE 5: N-Phényl-(5-hydroxy-1-méthylindol-2-yl)carboxamide

A un mélange, refroidi à 0°C, de 712 mg (2,84 mmol) de bromure d'aluminium et de 4,2 g (37,9 mmol) de thiophénol, sont ajoutés lentement 500 mg (1,9 mmol) du composé obtenu à l'exemple 4, préalablement dissous dans 5 ml de chlorure de méthylène. Dans deux intervalles d'une demi-heure sont alors ajoutés deux fois 1,5 éq. de bromure d'aluminium et 20 éq. de thiophénol. Après un total de 2 heures d'agitation à 0°C, la solution est jetée sur glace, acidifiée par de l'acide chlorhydrique 1N jusqu'à pH = 2 puis extraite au dichlorométhane. Après traitement habituel de la phase organique, le produit brut est purifié par chromatographie sur colonne de silice.
Rendement : 44 %.
Point de fusion : 195°C.

### EXEMPLE 6: N-Phényl-(5-éthylthio-1-méthylindol-2-yl)carboxamide

A un mélange refroidi à 0°C de 380 mg (2,84 mmol) de chlorure d'aluminium et de 2,3 g (37,9 mmol) d'éthanethiol, sont ajoutés goutte à goutte 500 mg (1,9 mmol) du composé obtenu à l'exemple 4, préalablement dissous dans 5 ml de chlorure de méthylène distillé sur pentoxyde de phosphore. Après 1 heure d'agitation à 0°C sont ajoutés à nouveau 1,5 éq. de chlorure d'aluminium et 20 éq. d'éthanethiol. Le mélange est agité encore une heure à 0°C puis jeté sur glace, traité par de l'acide chlorhydrique 1N puis extrait au dichlorométhane. Après traitement habituel de la phase organique et purification du produit brut par chromatographie sur colonne de silice, le composé attendu est obtenu.
Rendement : 30 %.
Point de fusion : 130°C.

Les exemples 7 et 8 sont préparés selon le mode opératoire décrit pour l'exemple 1 en condensant l'amine correspondante au composé souhaité.

### EXEMPLE 7: N-Phényl-(5-méthoxyindol-2-yl)carboxamide

Point de fusion : 191°C.

### EXEMPLE 8: N-(3-Méthoxyphényl)-(5-méthoxyindol-2-yl)carboxamide

Point de fusion : 183°C.

Les exemples 9 à 12 sont préparés selon le mode opératoire décrit pour l'exemple 1 puis de l'exemple 3 pour la O-déméthylation.

### EXEMPLE 9: N-Phényl-(5-hydroxyindol-2-yl)carboxamide

Point de fusion : 227°C.

### EXEMPLE 10: N-(3,4,5-Triméthoxyphényl)-(5-hydroxyindol-2-yl)carboxamide

Point de fusion : 220°C.

### EXEMPLE 11: N-Méthyl-N-phényl-(5-hydroxyindol-2-yl)carboxamide

Point de fusion : 193°C.

### EXEMPLE 12: N-Phényl-N-(n-octyl)-(5-hydroxyindol-2-yl)carboxamide

Point de fusion : 160°C.

Les exemples 13 à 17 sont réalisés selon le mode opératoire décrit pour l'exemple 5.

### EXEMPLE 13: N-(3-Chlorophényl)-(5-hydroxy-1-méthylindol-2-yl)carboxamide

Point de fusion : 187°C.

### EXEMPLE 14: N-Méthyl-N-(3-chlorophényl)-(5-hydroxy-1-méthylindol-2-yl) carboxamide

Point de fusion : 142°C.

### EXEMPLE 15: N-Méthyl-N-(3-trifluorométhylphényl)-(5-hydroxy-1-méthylindol-2-yl) carboxamide

Point de fusion : 158°C.

### EXEMPLE 16: N-Méthyl-N-(3-bromophényl)-(5-hydroxy-1-méthylindol-2-yl)carboxamide

Point de fusion : 144°C.

### EXEMPLE 17: N-(n-Butyl)-N-phényl-(5-hydroxy-1-méthylindol-2-yl)carboxamide

Point de fusion : 150°C.

### EXEMPLE 18: N-Phényl-N-(n-octyl)-(5-éthylthioindol-2-yl)carboxamide

Composé réalisé selon un mode opératoire identique à celui décrit pour l'exemple 6.
Point de fusion : 87°C.

En procédant de la même façon que pour les exemples 1, 2 ou 3, on obtient:

### EXEMPLE 19: 1-Méthyl-5-hydroxy-2-{4-[bis-(4-fluorophényl)méthyl]pipérazin-1-yl} carbonylindole

Point de fusion : 220°C.

### EXEMPLE 20: 1-Méthyl-5-hydroxy-2-[4-(2,3,4-triméthoxybenzyl)pipérazin-1-yl]carbonylindole

### EXEMPLE 21: 1-Méthyl-5-hydroxy-2-[4-(4-fluorobenzoyl)pipéridin-1-yl]carbonylindole

### EXEMPLE 22: 1-Méthyl-5-méthoxy-2-{4-[bis-(4-fluorophényl)méthylène]pipérazin-1-yl} carbonylindole

### EXEMPLE 23: N-(Quinol-3-yl)-(5-phénoxy-1-méthylindol-2-yl)carboxamide

### EXEMPLE 24: 1-Méthyl-5-benzyloxy-2-[4-(pyrimid-2-yl)pipérazin-1-yl]carbonylindole

### EXEMPLE 25: 1-Méthyl-5-(2-pyridyl)oxy-2-(4-benzylpipérazin-1-yl)carbonylindole

### EXEMPLE 26: 1-Méthyl-5-méthoxy-2-(4-phénéthylpipérazin-1-yl)carbonylindole

### EXEMPLE 27: 5-Hydroxy-2-{4-[bis-(4-fluorophényl)méthyl]pipérazin-1-yl} carbonylindole

### EXEMPLE 28: N-Cyclohexyl-N-méthyl-(5-méthoxyindol-2-yl)carboxamide

### EXEMPLE 29: 5-Méthoxy-2-(4-méthylpipéridino)carbonylindole

### EXEMPLE 30: 5-Benzyloxy-2-{4-[bis-(phényl)méthyl]pipéridino}carbonylindole

### EXEMPLE 31: N-Méthyl-N-(pyrid-2-yl)-(5-méthoxyindol-2-yl)carboxamide

### EXEMPLE 32: 5-Méthoxy-2-(4-n-propylpipérazin-1-yl)carbonylindole

### EXEMPLE 33: 5-(2,6-Diméthylpyrid-4-yl)-2-[4-(4-fluorobenzoyl)pipérazin-1-yl]carbonylindole

### EXEMPLE 34: 5-Benzyloxy-2-{4-[bis-(4-fluorophényl)méthyl]pipérazin-1-yl} carbonylindole

### EXEMPLE 35: 5-Hydroxy-2-[4-(2,3,4-triméthoxybenzyl)pipérazin-1-yl]carbonylindole

Point de fusion : 181°C.

Les exemples suivants sont préparés selon le mode opératoire décrit pour l'exemple 6.

### EXEMPLE 36: 1-Ethyl-5-éthylthio-2-{4-[bis-(4-fluorophényl)méthyl]pipérazin-1-yl} carbonylindole

### EXEMPLE 37: 1-Méthyl-5-méthylthio-2-[4-(phényl)pipérazin-1-yl]carbonylindole

### EXEMPLE 38: N-Phényl-N-(n-butyl)-(5-benzylthioindol-2-yl)carboxamide

### EXEMPLE 39: N-Benzyl-N-méthyl-(5-éthylthioindol-2-yl)carboxamide

### EXEMPLE 40: 5-Benzylthio-2-[4-(4-fluorophényl)pipérazin-1-yl]carbonylindole

### EXEMPLE 41: N-(n-Butyl)-N-phényl-(5-hydroxy-1-méthylindol-2-yl)méthylamine

2 mmoles du composé obtenu dans l'exemple 17 sont chauffés à reflux sous atmosphère d'azote en présence de 5 mmol d'hydrure double de lithium et d'aluminium dans 35 cm³ d'éther éthylique. Après refroidissement, hydrolyse, élimination par filtration des insolubles minéraux et séchage sur sulfate de sodium, le produit brut est purifié par chromatographie sur colonne de silice.
Rendement : 75 %

En utilisant un mode opératoire identique à partir des carboxamides appropriés, les amines suivantes sont obtenues:

### EXEMPLE 42: 1-Méthyl-5-hydroxy-2-{4-[bis-(4-fluorophényl)méthyl]pipérazin-1-yl} méthylindole

Composé obtenu à partir du composé de l'exemple 19

### EXEMPLE 43: 1-Méthyl-5-méthoxy-2-(4-phénéthylpipérazin-1-yl)méthylindole

Composé obtenu à partir du composé de l'exemple 26.

### EXEMPLE 44: N-(n-Butyl)-N-phényl-(5-hydroxy-1-méthylindol-2-yl)thiocarbonylindole

17 mmoles du composé de l'exemple 17 sont dissoutes dans 125 cm³ de toluène anhydre. 10,2 mmoles de réactif de Lawesson sont ajoutées et l'ensemble est porté à reflux pendant 6 heures. Après évaporation du solvant et purification sur colonne de silice, le produit du titre est obtenu.
Rendement : 90 %

De la même façon, on obtient :

### EXEMPLE 45 : 1-Méthyl-5-hydroxy-2-{4-[bis-(4-fluorophényl)méthyl]pipérazin-1-yl}thiocarbonylindole

Composé obtenu à partir du composé de l'exemple 19.

### EXEMPLE 46: 1-Méthyl-5-méthoxy-2-(4-phénéthylpipérazin-1-yl)thiocarbonylindole

Composé obtenu à partir du composé à partir du composé de l'exemple 26.

### EXEMPLE 47: N-(n-Hexyl)-N-phényl-(5-hydroxyindol-2-yl)carboxamide

Composé obtenu selon le mode opératoire décrit à l'exemple 12.
Point de fusion : 164°C

### EXEMPLE 48: N-(n-Hexyl)-N-phényl-(5-hydroxy-1-méthylindol-2-yl)carboxamide

Composé obtenu selon le mode opératoire décrit à l'exemple 17.
Point de fusion : 90°C

### EXEMPLE 49: 5-Hydroxy-1-méthyl-{4-[bis-(4-fluorophényl)méthylène]pipéridin-1-yl}carbonylindole

Composé obtenu selon le mode opératoire décrit à l'exemple 19.

### ETUDE PHARMACOLOGIQUE

### Exemple A: Etude du pouvoir protecteur de l'oxydation des LDL

La capacité des composés de l'invention à diminuer les proportions de LDL oxydées a été mesurée de la façon suivante : on réalise une incubation de 24 heures regroupant des LDL natives, un système Cu²⁺ générateur de radicaux libres et les composés à tester.
Les résultats sont obtenus après analyse du milieu par une technique chromatographique haute performance : la FPLC (Fast Protein Liquid Chromatography). Le pouvoir protecteur du composé testé est déterminé après comparaison du chromatogramme obtenu avec celui du témoin positif de référence : le probucol. Il apparaît clairement que les composés de l'invention ont un pouvoir protecteur très important. A titre de comparaison, pour une concentration de 10⁻⁵ M, le niveau de protection obtenu pour les composés de l'invention dépasse celui du probucol. C'est le cas notamment du composé décrit dans l'exemple 17 dont le pouvoir protecteur vis-à-vis d'un système oxydant des LDL est pratiquement total.

### Exemple B: Etude du pouvoir protecteur de l'oxydation des LDL (dosage du malondialdéhyde)

Des LDL humaines purifiées sont incubées en présence de sulfate de cuivre à la concentration de 5.10⁻⁶ M en l'absence ou en présence des composés à étudier.
L'activité des produits testés est évaluée par le calcul de la concentration réduisant de 50% (IC₅₀) la production de malondialdéhyde (MDA) par rapport aux expériences contrôles effectuées en l'absence de produit.
Les composés de l'invention sont remarquablement actifs dans ce test avec des IC₅₀ de 10⁻⁷ M très largement inférieures à celles déterminées pour le probucol.

### Exemple C: Etude ex vivo chez le lapin Watanabe

Des lapins Watanabe ont été traités une fois par jour par voie orale soit par le véhicule (groupe contrôle), soit par les composés de l'invention à la dose de 50 mg/kg/jour pendant 3 jours. Au troisième jour, 3 heures après le dernier traitement, les animaux sont sacrifiés et le sang est collecté sous acide éthylènediaminetétraacétique (EDTA). Les LDL (Low Density Lipoproteins) sont purifiées par ultracentrifugation et soumises à une oxydation par le sulfate de cuivre (5.10⁻⁶M). La peroxydation lipidique des LDL est appréciée, après différents temps d'incubation (de 2 à 48 heures) avec le sulfate de cuivre par la mesure de la formation de MDA (malondialdéhyde).
Les composés de l'invention montrent une réduction significative de la production de MDA comparativement au témoin (p < 0.001) traduisant ainsi une activité protectrice vis-à-vis de la peroxydation lipidique *ex vivo* après administration *per os*.

### Exemple D: Etude de l'activité antiperoxydante

L'action des composés utilisés selon l'invention, susceptibles de piéger les radicaux ·OH, a été étudiée d'une part, sur la peroxydation spontanée des lipides et d'autre part, sur la peroxydation induite par le système Fe²⁺ - ascorbate (10 µM - 250 µM), et ce, sur des homogénats de cerveaux de rats.

### a) Etude de la peroxydation lipidique spontanée

Lors de la mesure de la peroxydation lipidique spontanée, les homogénats de cerveau de rats sont placés en présence ou en absence des composés à tester durant 60 minutes à 37°C. La réaction est arrêtée à 0°C et le dosage du malondialdéhyde est effectué à l'aide d'acide thiobarbiturique. La peroxydation lipidique est déterminée par les substances réagissant avec l'acide thiobarbiturique exprimées en nanomoles de malondialdéhyde (MDA). Les concentrations des composés testés inhibant de 50 % la peroxydation du substrat sont calculées. Il est apparu que les composés de formule (I) utilisés selon l'invention possèdent une activité antiperoxydante particulièrement intense puisqu'ils possèdent une activité antiperoxydante nettement plus importante que le probucol et la vitamine E, qui est l'antioxydant naturel de l'organisme humain.

### b) Etude de la peroxydation lipidique induite

Lors de la mesure de la peroxydation lipidique induite, la méthodologie est identique à celle précédemment décrite à l'exception de l'addition à l'homogénat du système inducteur de radicaux : Fe²⁺ - ascorbate. Les substances de référence sont le probucol et la vitamine E. Les concentrations des composés testés inhibant de 50 % la peroxydation du substrat sont calculées. Par exemple, le composé de l'exemple 17 présente une IC₅₀ (MDA) égale à 5.10⁻⁷ M (Ratio probucol = 6).

### Exemple E: Etude de l'activité anti-inflammatoire

Les mesures sont réalisées sur granulocytes de lapins. Les granulocytes isolés sont stimulés in vitro par l'ionophore calcique (A 23187). Les leucotriènes B₄ libérés dans le milieu extracellulaire sont mesurés par Radio Immuno Assay (RIA). Les résultats (moyennes de 3 mesures indépendantes) sont exprimés en pourcentage d'inhibition par rapport au témoin.

A titre d'exemple, le composé de l'exemple 17 présente un pouvoir inhibiteur de 51% à 10 µM.

### Exemple F: Etude de l'activité anti-agrégante

Dans cette étude, les composés de l'invention (100 µg/ml) ont été testés dans le but de quantifier leur pouvoir d'inhibition du maximum non-réversible d'agrégation plaquettaire (plasma de lapin enrichi en plaquettes) induite par l'arachidonate de sodium (50 µg/ml). Ainsi, la concentration minimale active pour le composé de l'exemple 17 est de 30 µM.

### Exemple G : Composition pharmaceutique : comprimés

Formule de préparation pour 1000 comprimés dosés à 50 mg.
- Composé de l'exemple 1: 50 g
- Amidon de blé: 15 g
- Amidon de maïs: 15g
- Lactose: 65 g
- Stéarate de magnésium: 2 g
- Silice: 1 g
- Hydroxypropylcellulose: 2 g

## Revendications

1. Composés de formule (I) : 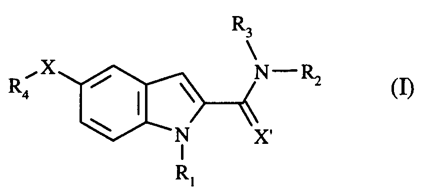 dans laquelle :
- R₁ est choisi parmi l'hydrogène et un radical alkyle linéaire ou ramifié, éventuellement substitué par un ou plusieurs groupements R₅, et comportant de 1 à 5 atomes de carbone,
- R₂ est choisi parmi l'hydrogène, un radical alkyle linéaire ou ramifié, éventuellement substitué par un ou plusieurs groupements R₅, et comportant de 1 à 12 atomes de carbone, et un radical cycloaliphatique comportant de 3 à 8 atomes de carbone éventuellement substitué par un ou plusieurs groupement R₅,
- R₃ est choisi parmi :
* un radical cycloaliphatique comportant de 3 à 8 atomes de carbone,
* un radical aryle, choisi parmi le radical phényle et le radical naphtyle,
* et un radical hétéroaryle choisi parmi le radical furyle, thiényle, thiazolyle, pyrrolyle, imidazolyle, pyridyle, pyrimidinyle, pyrazolyle, pyrazinyle, pyridazinyle, quinolyle, isoquinolyle, indolyle, benzofuranyle, benzo[b]thiényle et benzimidazolyle,
chacun des radicaux cycloaliphatiques, aryles et hétéroaryles pouvant être éventuellement substitués par un ou plusieurs groupements R₅,
- ou bien R₂ et R₃ forment ensemble avec l'atome d'azote auquel ils sont reliés un radical choisi parmi :
a) le radical de formule (a) : 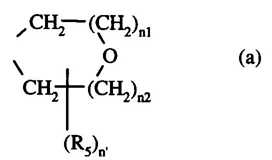
b) le radical de formule (b) : 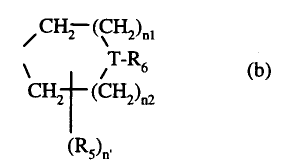
c) et le radical de formule (c) : 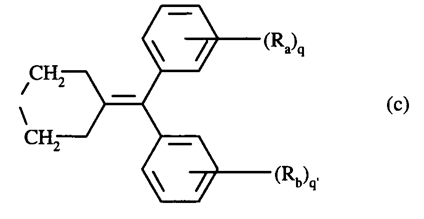
- R₄ est choisi parmi l'hydrogène, un radical alkyle linéaire ou ramifié, éventuellement substitué par un ou plusieurs groupements R₅, et comportant de 1 à 5 atomes de carbone, un radical aryle, hétéroaryle, choisis parmi ceux décrits pour le radical R₃, un radical arylalkyle et un radical hétéroarylalkyle dans lequel les radicaux aryle et hétéroaryle sont tels que définis précédemment et la chaîne alkyle comporte de 1 à 5 atomes de carbone en chaîne droite ou ramifiée, chacun étant éventuellement substitué par un ou plusieurs groupements R₅,
- R₅ est choisi parmi un halogène, un groupement hydroxy, nitro, cyano, alkyle, alkoxy, acyle, carboxy, alcoxycarbonyle, carboxamido, halogénoalkyle, amino, alkylamino et dialkylamino, les chaînes alkyles des groupements alkyles, alkoxy, acyles, alcoxycarbonyles, carbamoyles, halogénoalkyles, alkylamino et dialkylamino comportant de 1 à 5 atomes de carbone en chaîne droite ou ramifiée,
- R₆ représente soit l'hydrogène, soit un radical choisi parmi :
α) le radical 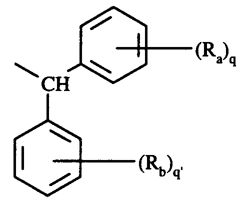 β) et le radical 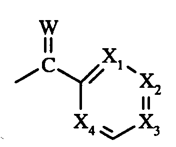
- X₁, X₂, X₃ et X₄ représentent indépendamment l'un de l'autre l'azote, le groupement CH ou le groupement C-Rₐ,
- Rₐ et R_{b}, identiques ou différents, sont choisis indépendamment l'un de l'autre parmi un halogène, un radical hydroxy, alkyle, alkoxy et halogénoalkyle, chacun contenant de 1 à 5 atomes de carbone en chaîne droite ou ramifiée,
- T est choisi parmi le groupement CH ou l'atome d'azote,
- W est choisi parmi O ou H₂,
- n₁ prend les valeurs 0, 1, 2, ou 3,
- n₂, dépendant de n₁, prend les valeurs entières comprises inclusivement entre 0 et (3-n₁),
- n' prend les valeurs de 0 à (n₁+n₂+2) (bornes incluses),
- m est choisi parmi 0, 1, 2, 3 et 4,
- q et q' prennent indépendamment l'un de l'autre les valeurs de 0 à 5 (bornes incluses),
- X est choisi parmi O ou S,
- X' est choisi parmi O, S ou H₂,
avec les restrictions suivantes :
- lorsque R₂ représente l'hydrogène alors R₃ ne peut pas représenter un radical phényle substitué en position 4 par une chaîne de nature oxy-2-hydroxy (ou alkoxy)-3-aminopropyle, ni un radical phényle substitué en position 2 par un radical carboxy,
- lorsque R₂ représente l'hydrogène, X le soufre et R₄ le radical para-nitrophényle, alors R₃ ne peut pas représenter un radical aryle éventuellement substitué,
- lorsque R₂ et R₃ forment ensemble avec l'atome d'azote auquel ils sont reliés un radical choisi parmi ceux de formules (a), (b) et (c) tels que définis précédemment, alors -X-R₄ ne peut représenter un radical -O-alkyle tel que défini précédemment,
leurs éventuels stéréoisomères, N-oxydes et sels d'addition à un acide ou une base pharmaceutiquement acceptables.

2. Composés selon la revendication 1, pour lesquels R₁ représente l'atome d'hydrogène, leurs stéréoisomères, N-oxydes et sels d'addition à un acide ou à une base pharmaceutiquement acceptables.

3. Composés de formule (I), selon la revendication 1, pour lesquels R₁ représente le radical méthyle, leurs stéréoisomères, N-oxydes et sels d'addition à un acide ou à une base pharmaceutiquement acceptables.

4. Composés de formule (I), selon la revendication 1, pour lesquels R₄ représente l'atome d'hydrogène, leurs stéréoisomères, N-oxydes et sels d'addition à un acide ou à une base pharmaceutiquement acceptables.

5. Composés de formule (I), selon la revendication 1, pour lesquels R₄ représente le radical méthyle, leurs stéréoisomères, N-oxydes et sels d'addition à un acide ou à une base pharmaceutiquement acceptables.

6. Composés de formule (I), selon la revendication 1, pour lesquels X représente l'oxygène, leurs stéréoisomères, et sels d'addition à un acide ou à une base pharmaceutiquement acceptables.

7. Composé selon la revendication 1, qui est le N-(n-butyl)-N-phényl-(5-hydroxy-1-méthylindol-2-yl)carboxamide, ainsi que ses sels d'addition à une base, pharmaceutiquement acceptables.

8. Composé selon la revendication 1, qui est le 1-méthyl-5-hydroxy-2-{4-[bis-(4-fluorophenyl)méthyl]pipérazin-1-yl}carbonylindole, ainsi que ses sels d'addition à un acide ou à une base, pharmaceutiquement acceptables.

9. Composé selon la revendication 1, qui est le N-(n-hexyl)-N-phényl-(5-hydroxyindol-2-yl)carboxamide, ses stéréoisomères, N-oxydes, ainsi que ses sels d'addition à une base, pharmaceutiquement acceptables.

10. Procédé de préparation des composés de formule (I) selon la revendication 1, caractérisé en ce que le produit de départ est l'acide de formule (II) : 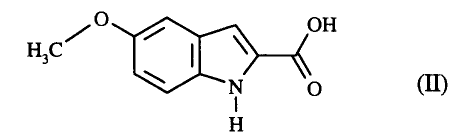 qui est transformé en chlorure d'acyle correspondant par un réactif approprié, par exemple le pentachlorure de phosphore, dans un solvant polaire, par exemple l'éther, puis est soumis à l'action d'une amine secondaire de formule (III) : 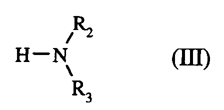 dans laquelle R₂ et R₃ sont tels que définis pour la formule (I),
afin d'obtenir l'amide de la formule (IV) : 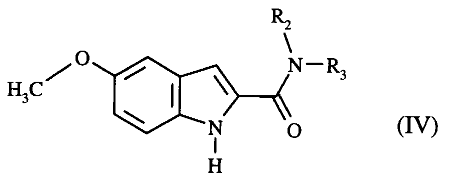 dans laquelle R₂ et R₃ sont tels que définis précédemment,
dont l'atome d'azote de l'indole est éventuellement alkylé par action successive d'un agent de déprotonation, comme l'hydrure de sodium, puis d'un agent alkylant de formule B-R'₁ dans laquelle B représente un atome d'halogène ou un groupement sulfate et R'₁ représente un radical alkyle linéaire ou ramifié, éventuellement substitué par un ou plusieurs groupements R₅, et comportant de 1 à 5 atomes de carbone, dans un solvant approprié, tel que le diméthylformamide, pour fournir les composés de formule (IV') : 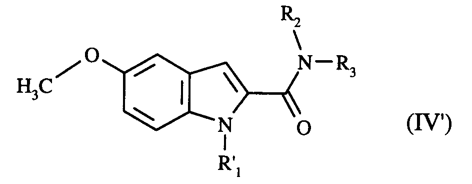 dans laquelle R'₁, R₂ et R₃ sont tels que définis précédemment,
l'ensemble des composés de formules (IV) et (IV') formant l'ensemble des composés de formule (Va) : 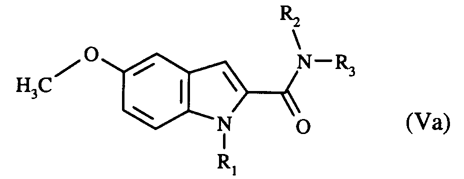 dans laquelle R₁, R₂ et R₃ sont tels que définis précédemment,
cas particulier des composés de formule (I), dans laquelle -X-R₄ représente le groupe méthoxy,
les composés de formule (Va) pouvant, si on le souhaite, être soumis à une réaction de O-déméthylation selon la méthode décrite par Fujita *et al*. (J. Org. Chem., 45, 4275, (1980)) par action d'un acide de Lewis et d'un alkylthiol, afin d'obtenir les composés de formule (Vb) : 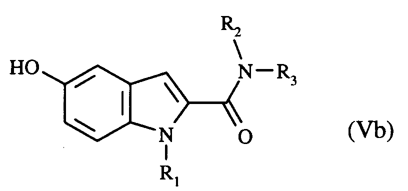 dans laquelle R₁, R₂ et R₃ sont tels que définis précédemment,
réaction conduisant également, selon les conditions opératoires, et en utilisant un alkylthiol de formule R'₄-SH, dans laquelle R'₄ prend toutes les valeurs possibles pour R₄, l'hydrogène excepté, aux composés de formule (Vc) : 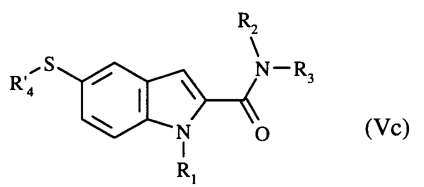 dans laquelle les substituants R₁, R₂, R₃ et R'₄ sont tels que définis précédemment,
les composés de formule (Vd) : 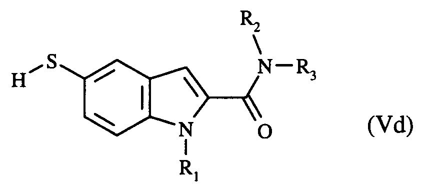 dans laquelle R₁, R₂ et R₃ sont tels que définis précédemment,
étant obtenus par débenzylation, par réduction chimique ou catalytique, des composés de formule (Vc') : 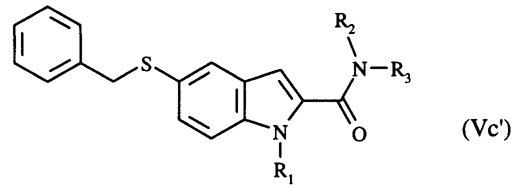 cas particulier des composés de formule (Vc) dans laquelle R'₄ représente le radical benzyle,
les composés de formule (Vb) étant éventuellement engagés dans une réaction classique d'éthérification pour fournir les composés de formule (Ve) : 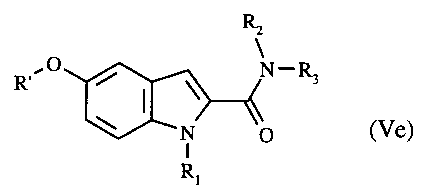 dans laquelle R₁, R₂ et R₃ sont tels que définis précédemment et R' représente un radical alkyle linéaire ou ramifié, éventuellement substitué, contenant de 2 à 5 atomes de carbone, un radical aryle, arylalkyle, hétéroaryle ou hétéroarylalkyle, choisis parmi ceux décrits pour R₄,
l'ensemble des composés de formules (Va), (Vb), (Vc), (Vd) et (Ve) formant l'ensemble des composés de formule (V) : 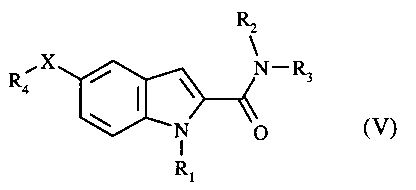 dans laquelle X, R₁, R₂, R₃ et R₄ sont tels que définis précédemment,
cas particulier des composés de formule (I) dans laquelle X' représente l'oxygène, qui sont soit :
- transformés, par traitement par le réactif de Lawesson, en thioamide de formule (V') : 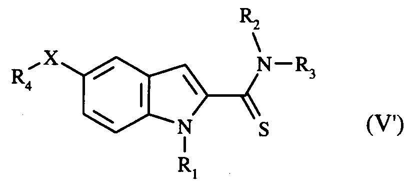 dans laquelle X, R₁, R₂, R₃ et R₄ sont tels que définis précédemment,
cas particulier des composés de formule (I) dans laquelle X' représente le soufre,
- soit réduits, à l'aide d'un agent réducteur, tel que l'hydrure double de lithium et d'aluminium. en solvant anhydre, tel que l'éther éthylique, en composés de formule (V'') : 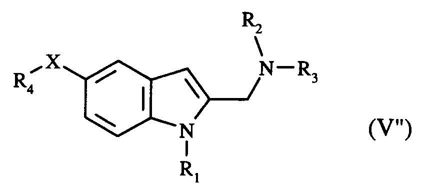 dans laquelle X, R₁, R₂, R₃ et R₄ sont tels que définis précédemment,
cas particulier des composés de formule (I) dans laquelle X' représente le groupement H₂,
l'ensemble des composés de formules (V), (V') et (V'') formant l'ensemble des composés de formule (I) qui sont purifiés et éventuellement séparés en leurs stéréoisomères par une technique classique de séparation et qui sont, si on le désire, transformés en leurs sels d'addition à un acide ou à une base pharmaceutiquement acceptables.

11. Compositions pharmaceutiques contenant comme principe actif au moins un composé selon l'une quelconque des revendications 1 à 9, seul ou en combinaison avec un ou plusieurs véhicules inertes, non toxiques et pharmaceutiquement acceptables.

12. Compositions pharmaceutiques, selon la revendication 11 exerçant une activité anti-oxydante spécifique des LDL et des lipides membranaires et utiles dans le traitement ou la prévention des affections dues ou reliées à ces phénomènes de peroxydation et notamment les désordres ischémiques cérébraux, rénaux ou cardiaques et les maladies métaboliques notamment l'athérome et l'artériosclérose, ainsi que l'inflammation.
